# EUROPEAN PATENT SPECIFICATION

(11) **EP 2 125 009 B1**
(45) Date of publication and mention of the grant of the patent: **16.05.2012**
(21) Application number: 07849664.3
(22) Date of filing: 06.06.2007
(51) Int. Cl.: A61K 39/12, A61P 17/06, A61P 17/00, A61P 19/02, A61K 35/76

(54) **MEDICINAL FORMULATION CONTAINING A COMBINATION OF HIV TYPE I AND HIV TYPE II**
MEDIZINISCHE FORMULIERUNG MIT EINER KOMBINATION VON HIV TYP I UND HIV TYP II
PREPARATION MEDICINALE CONTENANT UNE COMBINAISON DU VIH DE TYPE I ET DU VIH DE TYPE II

(30) Priority: 21.02.2007 IN MU03472007
(43) Date of publication of application: 02.12.2009
(73) Proprietor: Shah, Rajesh, Mumbai 400 089 MAH (IN)
(72) Inventor: Shah, Rajesh, Mumbai 400 089 MAH (IN)
(74) Representative: Williams, Paul Edwin
(86) International application number: PCT/IN2007/000229
(87) International publication number: WO 2008/102368

(56) References cited:
- WO-A1-95/15761
- DE-A1- 4 236 069
- DE-A1- 4 236 069
- FR-A1- 2 699 822
- FR-A1- 2 699 822
- IN-A- 371M UM2 005
- IN-A- 415M UM2 005
- DATABASE WPI Week 200602 Thomson Scientific, London, GB; AN 2006-013195 XP002621721, & IN 200 500 371 A1 (SHAH R N) 12 August 2005 (2005-08-12)
- DATABASE WPI Week 200602 Thomson Scientific, London, GB; AN 2006-013196 XP002621684, & IN 200 500 415 A1 (SHAH R N) 12 August 2005 (2005-08-12)

## Description

### Field of invention

This invention relates to medicinal formulations.

In particular, the present invention relates to a novel medicinal formulation for triggering an immune response in the body of a human being or animal that leads to therapeutic and prophylactic treatment of various immunologically mediated diseases.

### Definitions:

As used in the present specification, the following words and phrases are generally intended to have the meanings as set forth below, except to the extent that the context in which they are used indicates otherwise.

Immunologically mediated diseases are diseases where the immune response of an individual to a stimulus in an environment is altered.

There are three types of immunologically mediated diseases:
1. Immunosuppressive diseases: These are the diseases where the immune system is suppressed. Examples- HIV-AIDS or other opportunistic infections.
2. Hyper-responsive diseases: In these diseases, the body creates a hyper response to certain allergic substances. Example- Some individuals tend to become hypersensitive towards pollens and develop asthmatic conditions.
3. Autoimmune diseases: When a body encounters a foreign substance in its environment, it mounts an immune response against that substance to protect itself from potential harm. In order to do this effectively, it must be able to recognize what is self protein in order to respond to non-self or foreign protein. In autoimmune diseases, there is a failure to recognize some part of self tissues. Such recognition is restricted to a single protein or group of proteins. Such autoimmune reaction is restricted to a single organ or localized region or a generalized reaction in all over the body. The consequences may vary from minimal to catastrophic, depending on the extent to which the body is affected. In autoimmune disease pathologic signs are seen as a result of the autoimmune response. Frequently more than one autoimmune disease will be seen in the same animal, as well as an increased susceptibility to bacterial infection

HIV: Human immunodeficiency virus (HIV) is a retrovirus that causes Acquired Immunodeficiency Syndrome (AIDS), a condition in which the immune system fails to function, which results in life-threatening opportunistic infections. HIV is classified as a lentivirus from the family of Retroviridae.

HIV-I (HUMAN IMMUNODEFICIENCY VIRUS TYPE 1): It is a retrovirus isolated and recognized as the etiologic agent (cause) of AIDS. Most viruses and all bacteria, plants, and animals have genetic codes made up of DNA, which uses RNA to build specific proteins. The genetic material of a retrovirus such as HIV is single stranded, positive sense, enveloped RNA.

Upon entry in the target cell, the viral RNA genome is converted to doublestranded DNA by a virally encoded reverse transcriptase that is present in the virus particle. This viral DNA is then integrated into the cellular DNA by a virally encoded integrase so that the genome can be transcribed. Once the virus has infected the cell, two pathways are possible: either the virus becomes latent and the infected cell continues to function, or the virus becomes active and replicates, and a large number of virus particles are liberated that can then infect other cells.

HIV-II (HUMAN IMMUNODEFICIENCY VIRUS TYPE II): This virus is similar to HIV-I in structure and mechanism. It was first isolated in West Africa.

### Differences between HIV-I and HIV-II

Although HIV-I and HIV-II are similar in their structure, modes of transmission and resulting opportunistic infections, they differ in their geographic patterns of distribution.

Secondly, HIV-II is transmitted through sexual intercourse or from an infected woman to her fetus, while HIV-I can be transmitted through above stated ways as well as through infected blood to blood contact and use of infected needles and syringes. Thus, HIV-I infection spreads more rapidly than HIV-II infection.

Thirdly, the onset of AIDS is slower for those infected with HIV-II than HIV-I because those who are infected with HIV-II usually have a lower virus density in their bloodstream.

CD4 cells: CD4 cells are helper T cells which are infected by HIV. HIV infection leads to low levels of CD4 count. When the CD4⁺ T cell numbers decline below a critical level, body's cell-mediated immunity is compromised, and the body becomes more susceptible to opportunistic infections.

CD8 cells: CD8 are the suppressor T cells, which are lymphocytes (white blood cells), formed in the thymus and are part of the immune system. They have been found to be abnormal in persons with AIDS.

The CD4/CD8 ratio: The normal ratio of helper T cells (CD4 cells) to suppressor T cells (CD8 cells) is approximately 2. This is an indicator of the overall level of immune suppression or damage done by HIV. The lower the CD4/CD8 ratio, the worse the damage. The CD4/CD8 ratio is rarely less than 1.0 in HIV negative individuals, but may drop as low as 0.1 in patients with recent HIV infection or very advanced disease. The CD4/CD8 ratio will generally gradually decline over years of HIV infection in the absence of antiretroviral therapy.

Potency: Potency is a unit of dose in homeopathic medicine. Usually denoted as 'c' or centesimal potency, which means that the preparation has been made using the ratio of the original substance (1 part) to the vehicle 99parts (non-medicated substance, mostly ethyl alcohol (90 to 100%), normal saline or distilled water). It may be noted that there may be some variation in this ratio, which is not likely to alter the efficacy of the medicine. Alternatively, 1:10 ratio can be used which is a decimal potency, designated as `x'.

### Background and Prior Art:

HIV infection is considered as the most destructive pandemics that affect human beings. It is estimated that about 0.6% of the world's living population is infected with HIV. A third of these deaths are occurring in sub-Saharan Africa, retarding economic growth and increasing poverty. According to current estimates, HIV is set to infect 90 million people in Africa, resulting in a minimum estimate of 18 million orphans.

The current treatment entails antiretroviral therapy, which helps to certain extent but is very expensive and has adverse side effects. Attempts to produce vaccines for HIV infection have not been successful yet.

Use of nosodes in homeopathy: Nosodes are medicinal formulations used in homeopathy since the early part of the 19^{th} century. In preparation of nosodes, infinitesimal parts of a diseased tissues or organisms (bacteria or virus) are used by a method called potentisation, which entails mixing of 1 part (say 1ml of serum containing organisms) with 99 parts of non-medicated vehicle (water or alcohol) and exposing the content to powerful striking (about 10-12 times) on a hard surface, while holding the bottle tightly in one's hand. This process can also be done with the help of a mechanical device which may allow such mechanical hard-hitting. This process leads to production of a formulation, whose power is 1c or 1 centesimal potency. Such a procedure can be repeated upto 1 million times to obtain medicinal formulations of different potencies. For example, serial dilutions carried out 30 times provide a formulation of 30 c potency, 100 times provide 100 c potency and 200 times provide 200c potency.

Nosodes prepared from various diseased products and infected disease materials have been found to be useful in homeopathic practice. Despite the fact that they are prepared from various infective agents such as Mycobacterium tuberculosis, Rabies virus and the like, they are free from side effects and their safety is well established.

The preparation of nosodes derives from *homotoxicology,* a type of homeopathic therapy created by Hans-Heinrich Reckeweg in Germany in the first part of 18th century.

Homeopathic medicine, since its inception under Hahneman at the beginning of the 19th century, follows the principle of "infinitesimals." From this notion, the diseased products in nosodes are in very minute and diluted forms. Thus, nosodes are not harmful.

A 'nosode' is comparable with an "vaccine" in the sense that its purpose is to induce some immunity the specific infection. More than that, the nosodes have shown much broader application whereby they seem to trigger broad-spectrum immunological changes, hence altering the course of many diseases, remaining not just restructured to the disease from where it has been sourced.

Another major advantage of a nosode is, of course, the extremely small quantity of antigenic material in a nosode as compared to a vaccine.

In the case of nosodes from bacteria, viruses or diseased tissues, the preparation introduces molecular imprints of possible antigens and other constituents of the pathological agent to the immune system. The working of a nosode is based on the fact that the immune system is induced to develop a defense mechanism which is effective against variety of antigens with this kind of molecular imprint, without being exposed to the virulence of the living agent.

Nosodes are also used as inter-current remedies in the treatment of chronic diseases.

### Objects of the invention:

This invention provides a novel, broad spectrum immunity enhancing, immune-modulating, adaptogenic and effective medicinal formulation for the treatment of immunologically mediated diseases selected from psoriasis and rheumatoid arthritis.

Preferably, the medicinal formulation can be administered by oral route.

Preferably, the medicinal formulation is reproducible and contains precisely defined antigenic material.

Preferably, the medicinal formulation is safe, free from side-effects and economic.

This invention provides a homeopathic formulation which is effective for treatment of psoriasis and rheumatoid arthritis.

Preferably, the medicinal formulation reduces levels of various cytokines such as Interleukin-8 (IL 8), Tumor Necrosis Factor alpha (TNF α), interferon-γ (IFN-γ) which are important mediators of immunological diseases such as Psoriasis and Rheumatoid Arthritis.

This invention also provides a method for preparation of the formulation which preferably is easily reproducible, economic and does not require complicated process and equipment.

### Summary:

A novel medicinal formulation claimed for the treatment of said autoimmune diseases, comprises a serially diluted and potentised HIV Type I and HIV Type II virus combined together. The preparations are made from the sera of patients who were positive for HIV Type I and HIV Type II, with laboratory established diagnosis.

The above said formulation is prepared by a process of diluting and potentising the respective substances combined together, with appropriate amount of vehicle to obtain a primary dilution of 1 c potency. Each of the serially diluted substances are 1c dilutions of the original HIV Type I and Type II combination in the vehicle, each 'c' representing a dilution of the previous stage in the vehicle where the proportion of the substance to vehicle is in the range of 1:99 to 50:50. The serial dilution is in the range of 1c to 1million c.

Potentisation is effected by holding a bottle containing the formulation in a closed fist and striking the fist on a hard surface repeatedly at a regular frequency or by exercising similar powerful strokes using a mechanical device which can strike the bottle containing the formulation on a hard surface.

The medicinal formulation according to this invention may be administered alone or in combination with other nosodes or other immunomodulatory substances of natural or synthetic origin or with other old/new homeopathic preparations or with health improving substances such as vitamins, antioxidants, flavonoids and the like.

### Description

Co-pending Indian applications no. 371/MUM/2005 and 415/MUM/2005 filed by the inventor, describes nosodes prepared from HIV-I and HIV-II alone respectively. According to these inventions, the most suitable sources for the HIV type I and II nosodes are the body fluids of patients who were detected positive for HIV type I and II respectively and they should be negative for other similar infections such as Hepatitis C, herpes and the like.

However, further experimentation in accordance with this invention shows that the combination of HIV type I nosode with HIV type II nosode is more effective than the individual preparations.

Therefore, particularly envisaged in accordance with this invention is a safe medicinal formulation containing serially agitated dilutions of mixture of antigens from strains of HIV type I and HIV type II.

The formulation of this invention referred to as HIV combination, is the combination of HIV type I and HIV type II and has been found to be effective in the treatment of autoimmune diseases selected from psoriasis and rheumatoid arthritis.

There is no such known therapeutic measure comparable with the HIV combination prepared from the HIV type I and II virus; used for the treatment of said autoimmune diseases.

The exact mechanism of its action is required to be explored. However, the HIV combination seems to be working by triggering specific immune response in the body and general immune response whereby it could help various immunologically mediated diseases.

The HIV combination has also been found to be effective for various ailments. This combination may be administered repeatedly and in same or different potency (power or dose) over the period of time, without any adverse effects.

In accordance with this invention, there is also provided a method of production of the medicinal formulation comprising the following steps:-

### Step 1

### Collection of body fluids containing HIV virus:

A sample of serum of a patient who is HIV type I positive is collected from a reputed laboratory. A second sample is obtained of another patient who is positive for HIV type II. It is made sure that both the samples are positive only for the respective HIV types and they are not positive for the other HIV type.

It is also confirmed that both the samples are negative for other related viral infections such as Hepatitis C and Herpes genitalis. The samples are labeled as HIV type I and HIV type II respectively. It may also be noted that the preparations have been made using the serum, which contains the respective virus; and not from the blood, as frank blood will have bacteria, immunocytes, all blood cells, cytokines, etc. besides the virus.

It may also be noted that this initial procedure of the use of the infected serum from the patient may be replaced by using culture of HIV virus, whenever it may be possible to do so in future. At this time, there is no facility to have a culture of HIV virus.

### Step 2

### Preparation of HIV combination

0.5-1.5 milligram of HIV type I serum is taken in a suitably sized vial and mixed with 0.5-1.5 milligram of HIV type II serum. The two sera are taken in equal quantity and referred to as 'Mixed HIV substances'. To this 'Mixed HIV substance' is added 80-120 drops of distilled water.

The vial thus prepared in step 2 is labeled as 'HIV combination'. It may be noted that any minor variation in the serum quantity (1ml or more or less) and in the quantity of distilled water (100 drops or more or less) would neither change the efficacy of the ultimate product nor would it alter the originality of the product. Also, in this case, distilled water is considered as non-medicated vehicle, which may be replaced with any non-medicated substances such as ethyl alcohol or any such substance, which may not alter the effect of the original substance.

### Step 3

i. The vial labeled 'HIV combination' is held firmly in a closed fist (taking all aseptic precautions) and struck very hard on a hard surface from distance of about two feet. This procedure is repeated 10 times. This simple procedure leads to very vigorous shaking of the bottle content. This bottle is labeled as 'HIV combination - 1c', (1c = 1 centesimal), where 1c denotes the first level of power or dose of the preparation.
ii. One drop from the vial labeled as 'HIV combination - 1c' is now mixed with about 99 drops of alcohol or distilled water, in another vial. This new vial is now exposed to the same procedure of vigorous shaking as above, 10 times and the resultant vial is now labeled as 'HIV combination - 2c'. It may be noted that the original substance may be either one drop or more or less, which may not be claimed as originality of the product.

It may be noted the manual procedure of vigorous shaking by giving powerful strokes may be replaced by any suitable mechanical (or electromechanical) device which might do a similar job, saving human effort involved in the process. Also, any use of such a device may not be claimed as originality of the product, as it is just the means of procedure.

Also, any variation in the number of strokes from 10 to 100 or more or less, also would not add to any originality in the procedure.

### Step 4

i. The procedure is repeated to prepare HIV combination- 3c, HIV combination- 4c...HIV combination- 100c... HIV combination- 1000c... HIV combination- 1 million c and more.

The final products, preparations from two different virus sources are now ready for use in future.

These preparations are in incredibly minute dose, which are devoid of any virus or any disease-producing properties of the original organisms. These preparations are safe and free from known side effects.

It may also be noted that the homeopathic medicines thus prepared in the 15c or more potency, the scientists have not been able to find any molecule of the original substance when examined with the latest electron microscope. In other words, such preparations beyond 15c potency are free from any toxicity.

It may be noted that several toxic substances such as arsenic, potassium iodide, hydrocyanic acid, snake venom and the like are in use in homeopathy since over 100 years and they have been found to be free from their original toxicity. Such non-toxic products are legally sold over the counter in most countries.

It may be noted that the proportion of mixing of primary serum with the vehicle can range from 1:99 to 50:50. Preferably 1ml of the primary culture is mixed with 90 ml of the vehicle.

Although the dilution represented by the term 'c' means a dilution of 1:99, for the purposes of this specification the term 'c' is deemed to mean any dilution in the range of 1:99 to 50:50

### The HIV combination:

The inventor of the formulation experimented with the individual components in several patients. However, in a study, it was observed that when used in combination of two identical (or different) potencies, the results were much better. That is, instead of using HIV type I 50c alone, if it was combined with HIV type II 50c, or similar potency; the results were faster and better.

Based on this experience, the HIV combination was prepared. It may be noted that there may be several combinations and permutations possible for developing different potencies while using the HIV combination.

### About the potency:

Homeopathic medicines prepared from the diseased body tissues or organisms are prescribed to patients in various potencies, as per the well defined parameters of potency selection. Some of the parameters may be described in brief here under:
1. **Age**: Younger patients with higher susceptibility may be prescribed medium to high potency such as 30c to 1000c.
2. **Functional pathology:** Patients with functional disorders are prescribed medium to high potency.
3. **Structural pathology:** Patients with structural pathology are prescribed low potency to start with.
4. **Nosode:** When used as a nosode, 30c potency is the ideal in it can be stepped up slowly.
5. **Response:** In case there is no response or the remedy stops acting, the potency can be stepped up from 30c to 50c to 100c to 500c.
6. **Active pathology:** In case of active pathology such as tubercular cavities or ulcers, higher potencies should be avoided.
7. **Susceptibility:** Higher the susceptibility, higher the potency.
8. Newer medicines in their initial evaluation period are better prescribed in 30c potency, if they are prepared from organism, venoms or any toxic source.

### Serial Dilution and Potentisation:

Further to the procedure explained above, it may be noted as below. Potentisation is a mathematico-mechanical process for rendering inert or poisonous antigen containing pathological residues, to a state of physical solubility, physiological assimilability so as to enhance their therapeutic activity and harmlessness, for use as a healing remedy.

The primary objective of potentisation is to reduce all substances designed for therapeutic use to "a state of approximately perfect solution or complete ionization, which is fully accomplished only by infinite dilution." (Arrhenius.)

Each resulting diluted substance is potentised typically by stroking by holding the bottle in a closed fist and striking the fist on a hard surface repeatedly at a regular frequency or by exercising similar powerful strokes using a mechanical device which can strike a bottle on a hard surface. Such strokes are given about 10 times.

### Advantage and Application of the new formulation HIV combination:

These formulations are prepared from the specific HIV virus types but they lack the viral toxicity.

Preliminary clinical investigations have shown that the formulations prepared according to the above process help to retain the capacity to induce immune response in the body, which helps in treating immunologically mediated diseases selected from psoriasis and rheumatoid arthritis.

HIV type I and type II sera were procured from Hindusabha Hospital, Ghatkopar East, Mumbai, India; of patients who were respectively HIV type I and type II positive.

### Note on the combination of HIV type I and Type II:

The investigator, who also has patent pending for HIV type I as well as for HIV type II preparations, has experimented with both the versions separately in a series of cases. The results were good. However, when the investigator carried out a study whereby both the preparations were used in combination, they produced much better and more predictable results. Logically, the use of both the preparations offers a wider antigenic action. This leads to a broader immune reaction leading to superior applicability in terms of effective and faster results.

### Case 1

A 42-year old male, reported of suffering from psoriasis for 4 years. During this period, he developed mild to moderate lesions. The conventional treatment did not show any remission.

The formulation of HIV Type I was given in 100 c potency. Following this for about 1 month, the patient recovered 25-30%. Later aggravation was continued for about 4 months. HIV Type I was stopped and the patient was started on a HIV combination in 50 c potency, which showed a good response.

### Case 2

A 40 year old male was affected with psoriasis for 15 years. The body had moderate to extensive lesions. He attempted conventional treatment which did not show any remission.

He was prescribed HIV Type I, which was given in 50 c potency. An initial withdrawal of steroid induced a temporary intensification of symptoms for 2 months. HIV Type I was continued for 6 months for which the patient showed 75% recovery. During another 8 months of continuation with HIV combination in 30 c potency, the patient improved and showed complete recovery.

### Case 3

A 29 year old male was affected with psoriasis for 10 months. The body had severe lesions on palms and soles. He attempted for the conventional treatment which did not show any improvement.

He was prescribed HIV Type 1, which was given in 50 c potency. An initial withdrawal of steroid induced aggravation for a month. HIV Type I was continued for 6 months for which the patient showed 75% recovery. During another 8 months of continuation with HIV combination in 100 c potency, the patient improved and showed complete recovery.

### Case 4

A male aged 44 years presented with a history of psoriasis with extensive lesions for 7 years. Remission occurred twice.

He was prescribed HIV Type I, which was given in 35 c potency. The medicine was continued for 2 months and he improved 70%. Further treatment for 8 months with HIV combination in 100 c potency resulted in full recovery with no reoccurrence even for a year.

### Case 5

A male aged 47 years presented with a history of psoriasis with mild lesions for 2 years. He attempted for the conventional treatment which did not show any remission.

He was prescribed HIV Type 1, which was given in 50 c potency. The medicine was continued for 7 months and he was improved to 70%. Later, the treatment was discontinued and HIV combination was administered for 6 months in 100 c potency. There was 100% improvement and no reoccurrence.

### Case 6

A male aged 38 years presented with a history of psoriasis with large lesions for 5 years.

He was prescribed HIV Type I, which was given in 35 c potency. The medicine was continued for 12 months and he was improved to 25-30%. Later, the complaints kept appearing and disappearing; therefore HIV combination was given for 6 months in 100 c potency. Excellent results were seen and there was 90% reduction in the lesion.

### Case 7

A female aged 37 years presented with a history of psoriasis with moderate lesions for 4 years. The conventional treatment did not show any result.

She was prescribed HIV Type 1, which was given in 35 c potency. Afterwards, the potency was increased subsequently to 40 c and 50 c potency. After an initial aggravation for a month, the patient recovered completely in a period of 12 months but there was remission after 6 months. Therefore, HIV combination was administered in 100 c potency. Recovery was effected on 1 month and no remission occurred there after.

### Case 8

A female aged 65 years presented with a history of psoriasis with moderate lesions for 8 years. The conventional treatment did not show any result.

She was prescribed HIV Type I, which was given in 35 c potency. Afterwards, the potency was increased subsequently to 40 c and 50 c potency. The patient recovered 80% after 5 months of treatment. The treatment was continued with HIV combination in 100 potency for 2years and then concluded as she recovered almost completely.

### Case 9

A male aged 28 years presented with a history of psoriasis with extensive lesions for 10 years. He attempted for the conventional treatment which did not show any remission.

He was prescribed HIV Type I, which was given in 50 c potency. Afterwards, the potency was changed subsequently to 80 c, 40 c and 35 c potency. The medicine was continued for 4 months and he was improved to 25%. Later, the treatment was continued with HIV combination in 30 c potency for another 1 year, which recovered him almost 90%. The recovery was much faster after administering the HIV combination.

### Case 10

A female aged 24 years presented with a history of psoriasis with moderate lesions for 3 months. She attempted for the conventional treatment which did not show any remission.

She was prescribed HIV Type I, which was given in 35c potency. The medicine was continued for 6 months and she was improved to 50%. Thereafter she was *Status quo.* Treatment was continued with HIV combination in 50 c potency, which showed significant improvement with almost 90% recovery. Subsequently she was given the phototherapy as well.

### Case 11

A male aged 27 years presented with a history of psoriasis with extensive lesions for 4 years. The conventional treatment did not show any remission. He was prescribed HIV Type II, which was given in 35 c potency. Afterwards, the potency was changed subsequently to 200 c for twice a day. The treatment was continued for 5 months and he was recovered almost 80%. After that he was reported *status quo.* Further treatment was effected with HIV combination in 300c, with complete remission.

### Case 12

A 22 year old male was affected with psoriasis for 3 years. The body was reported to have extensive lesions. He attempted conventional treatment which did not show any remission.

He was prescribed HIV Type II in 30c potency along with HIV Type I in 30c initially. A withdrawal of steroid induced aggravation, which was controlled later with HIV Type II doses in 300c. The treatment was continued for 7 months and he was recovered almost 80%. Treatment was started with HIV combination in 200c with complete recovery after 3 months. The recovery was much faster after administering the HIV combination.

### Case 13

A male aged 44 years presented with a history of psoriasis with mild lesions for a year. He attempted conventional treatment which did not show any remission.

He was prescribed HIV Type II, which was given in 200 c potency for twice a day. The treatment was continued for 12 months and he was recovered almost 70%. Treatment was started with HIV combination in 300 c and he recovered fully in 6 months. The recovery was much faster after administering the HIV combination.

### Case 14

A female aged 30 years presented with a history of psoriasis with extensive lesions for 15 years. She attempted conventional treatment which did not show any remission.

She was prescribed HIV Type II, which was given in 35 c potency. Afterwards, the potency was increased subsequently to 50 c, 80 c and 200 c potency. The medicine was continued for 10 months and she was recovered almost 60%. Further treatment was effected with HIV combination in 50c and 200c potency. Recovery was seen almost 90-95%.

### Case 15

A 28 year old male was affected with psoriasis for 10 years. The body had extensive lesions. He attempted conventional treatment which did not show any remission.

Initially, he was prescribed HIV Type I, which was given in 50c, 80c, 40c and 35c potency. He improved to 25% after 4 months of treatment. Later, he was given was HIV Type II in 30c, which continued for another 1 year and he recovered almost 80%. Later he was given the HIV combination in 50 c which resulted in almost 100% recovery.

### Case 16

A 25 year old female was affected with psoriasis for 6 years. The body was reported to have moderately severe lesions. She attempted for the conventional treatment which did not show any remission.

Initially, she was prescribed HIV Type II, which showed some improvement. Later, she was given HIV combination in 30c potency. Patient had an improvement of 20-25% with HIV Type 2 alone. But the combination resulted in 80% recovery.

### Case 17

A 59 year old male was affected with psoriasis for 5 years. The body was reported to have moderate lesions. It was also reported that she had attempted for homeopathic medicines in past. The conventional medicines did not show any remission.

Initially, he was prescribed HIV Type II in 35 c potency, which had shown some improvement. Later, he was given HIV combination in 50c potency. Patient had reported to show an improvement of 20-25% with HIV Type II alone but the combination proved 100% recovery.

### Case 18

A 35 year old female was affected with psoriasis. The presence of psoriasis was recently diagnosed. The body was reported to have mild lesions. The conventional treatment did not show any remission.

Initially she was prescribed traditional homeopathic medicines. Later, she was given was HIV Type II in 50c and 100c potency for which she showed some improvement. After that she was *Status quo.* HIV combination in 50 c potency was tried which was increased to 300 c for 3 months, which resulted in full recovery.

### Case 19

A male aged 28 years presented with a history of psoriasis with moderate lesions for a year. He was under homeopathic treatment when Psoriasis was diagnosed. The conventional treatment did not show any remission.

Initially he was prescribed traditional homeopathic medicines. Later, he was given was HIV Type II for which he showed slight improvement initially. Later there was no response reported to the treatment; therefore HIV combination in 300c potency was tried for 6 months with almost 80-90% recovery. The recovery was much faster after administering the HIV combination.

### Case 20

A male aged 55 years presented with a history of psoriasis with extensive lesions for 20 years. The conventional treatment did not show any remission. Initially he was prescribed HIV Type I for which he showed slight improvement initially. Later there was no response reported to the treatment. Subsequently he was given HIV Type II in 50 c potency. The patient did not follow up thereafter for a while and presented with fresh lesions. HIV combination in 30c, 50c, 200c was tried with significant recovery.

### Case-21

A 34-year old male reported of suffering from psoriasis for one and half months. During this period, he developed moderately severe lesions. The patient also reported to have similar complaints 7 years ago. HIV combination was given in 50 potency. Following this for about 2 months, the patient recovered 90-95%. Then he continued for next 5 months and he was almost completely recovered.

### Case-22

A male aged 30 years presented with a history of psoriasis with mild lesions since 8 months. He had tried the conventional treatment but no remission was found.

He was prescribed HIV combination in 50 potency. Later, the potency was increased to 100c, 200c and 300c subsequently. In a period of 12 months, he reported an overall recovery of 60%. However, he discontinued the treatment after that.

### Case-23

A 22 year old male was affected with psoriasis for about a month. The body was reported to have mild to moderate lesions. He attempted for the conventional treatment which did not show any recovery.

He was prescribed HIV combination, which was given in 35c potency. Afterwards, the potency was increased to 200c. A temporary intensification of symptoms was observed for 6 weeks after that he started improving. The treatment was continued for 9 months and then concluded as he recovered almost completely.

### Case-24

A 21 year old male was reported to have psoriasis for 15 years with the history of masked lesions. The conventional treatment did not show any remission. He was prescribed HIV combination, which was given in 50c potency. Afterwards, the potency was increased to 200c. The treatment was continued for 9 months and then concluded as he recovered almost completely

### Case-25

A 44 year old male was reported with psoriasis for 13 years with the history of moderate lesions. A remission occurred once for 5 years during the conventional treatment.

He was prescribed HIV combination, which was given in 50c potency. Then he continued for next 3 months and he was almost 75% recovered.

### Case-26

A male aged 35 years presented with a history of psoriasis with extensive lesions for 8 years. The conventional treatment did not show any remission. He was prescribed HIV combination, which was given in 200c potency. The medicine was continued for 3 months and he was improved to 90%.

### Case-27

A 56 year old male was reported with psoriasis for 25 years with the history of extensive lesions. He was on conventional treatment during which spontaneous remissions occurred 2-3 times. The remissions lasted for 1-2 months.

Later, he was prescribed HIV combination, which was given in 200c potency. The medicine was continued for 6 months and he was improved to 75-80%.

### Case-28

A female aged 38 years presented with a history of psoriasis with extensive lesions for 2 years. The conventional treatment did not show any remission. She was prescribed HIV combination, which was given in 50c potency. The treatment was continued for 5 months and then concluded as she recovered almost completely.

### Case-29

A female aged 30 years presented with a history of psoriasis with moderate lesions for 15 years. She had tried the conventional treatment but no remission was found.

She was prescribed HIV combination in 50c potency. Later, the potency was increased to 100c subsequently. In a period of 10 months, she reported an overall recovery of 90%.

### Case-30

A 5 and ½ year old male presented with a history of psoriasis with moderate lesions for 2 years. He was on conventional treatment during which spontaneous remissions occurred 2-3 times. The remissions lasted for about 2 weeks.

He was prescribed HIV Type I in 35c potency, which resulted in 75 % recovery within a month. When his complaints started exacerbating, the HIV combination was given in 300 c potency. The treatment was continued for another 3 months and then concluded as he recovered almost completely.

### Comparative Case-1

Six years old female patient presented with extensive eczema all over the body since the age of one. She had had all conventional medications with temporary improvement. She was given conventional homeopathic treatment such as *Sulphur, Graphites, Psoriasis,* etc. by earlier homeopath without any significant improvement. She was administered the HIV combination 100c, thrice a day for a month. There was rapid improvement of about 40%. The treatment was continues for eight weeks to achieve 60% improvement. The treatment was continued for six more months and she was over 90% better.

### Comparative Case-2

Three years old male child presented with Molluscum Contagiousa (a viral infection) affecting his face, chest and hands since three months. He was put on the HIV combination 100c, thrice a day for a month. All his lesions disappeared.

### Case-31

Mrs. A, 45 years old female was treated for Rheumatoid Arthritis for over one year, with partial improvement with conventional homeopathic treatment. She was then administered the HIV combination 200c, thrice a day for six weeks. She dramatically improved. The treatment was continued for over one year to achieve a remission.

### Comparative Case-3

Sixty four years old male patient was under care for the treatment of Lichen Planus since six years. He was put on the HIV combination 50c, thrice a day for two months to achieve over 50% improvement.

### Comparative Case- 4

Six confirmed cases of HIV positive, with low CD4 count and moderate HIV viral load, have been prescribed the HIV combination 50c. Their progress and results are in the process of evaluation.

### Application and usage of HIV TYPE I and HIV TYPE II combination:

The said preparation of combination is found to be useful and its scope requires further exploration for the following conditions:
1. Various immunologically mediated diseases like Psoriasis, Eczema, allergic disorders, auto-immune diseases and the like.
2. Viral infections such as Herpes genitalis, Herpes zoster, AIDS, molluschum contagiosa, and the like.
3. Therapeutic and prophylactic use in cases of HIV infections and AIDS
4. Collagen diseases
5. Various other chronic diseases

### References:

1. J.T. Kent's Philosophy
2. M.L. Dhawale's Principles and Practice of Homoeopathy

## Claims

1. A medicinal formulation for use in the treatment of autoimmune diseases selected from psoriasis and rheumatoid arthritis, said formulation comprising, combined serially diluted and potentized HIV TYPE I virus and HIV TYPE II virus, said dilution being effected in a vehicle selected from a group consisting of distilled water or ethyl alcohol and mixture of distilled water and ethyl alcohol.

2. A formulation for use as claimed in claim 1, wherein the substances are obtained from sera, blood and body fluids of infected persons.

3. A formulation for use as claimed in claim 1, wherein each of the serially diluted substances are 1c dilutions of the original mixed HIV TYPE I and II substances in the vehicle, each 'c' representing a dilution of the previous stage in the vehicle where the proportion of the substance to vehicle is in the range of 1:99 to 50:50.

4. A formulation for use as claimed in claim 1, wherein the serial dilution is in the range of 1c to 1 million c.

5. A process for making a formulation as claimed in claims 1 to 4, comprising the following steps
i. obtaining predetermined quantity of HIV TYPE I substance,
ii. obtaining predetermined quantity of HIV TYPE II substance,
iii. mixing equal quantity of HIV TYPE I and HIV TYPE II,
iv. diluting mixture in step (iii) with appropriate amount of vehicle to obtain a primary dilution of 1 c potency,
v. serially diluting with potentization, the primary dilution of 1c potency in step (iv) with the vehicle in a ratio of original dilution to vehicle in the range of 1: 99 to 50:50 to obtain diluted and potentized HIV combination of 1c to 1 million c and more.

6. A process for making a formulation as claimed in claim 5, wherein potentization is effected by stroking by holding a bottle containing the diluted culture in a closed fist and striking the fist on a hard surface repeatedly at a regular frequency or by exercising similar powerful stroke using a mechanical device which can strike a bottle on a hard surface.

7. A process for making a formulation as claimed in claim 6, wherein strokes are given about 10 times.

8. A process for making a formulation as claimed in claims 6 or 7, wherein the bottle containing the preparation for stroking for potentization is a securely stoppered glass bottle.

9. A process for making a formulation as claimed in claims 6 to 8 in which a mechanical device is used to exert a force of at least 6 dynes rhythmically at a frequency of 10 strokes in two minutes.

## Patentansprüche

1. Ein Rezeptur medizinisch, die in der Behandlung der Autoimmunerkrankungen die aus Psoriasis und rheumatoider Arthritis verwendet werden, wobei das Rezeptur, die kombinierte, seriell verdünnte und potenzierte Humanes Immunodefizienzvirus Typ 1 (HIV-1) und Humanes Immunodefizienzvirus Typ 2 (HIV-2) umfaßt, wobei das Rezeptur bei einem Substanzträger, das aus eine Gruppe aus destilliertes Wasser und Ethylalkohol oder au seiner Mischung von destilliertes Wasser und Ethylalkohol ausgewählt ist, ausgeführt wird.

2. Ein Rezeptur zur Verwendung gemäß Anspruch 1, wobei die Substanzen aus Seren, Blut und Körperflüssigkeiten der infizierten Menschen gewonnen werden.

3. Ein Rezeptur zur Verwendung gemäß Anspruch 1, wobei jeder Substanzen 1c Verwässerung der ursprünglichen gemischten Substanzen der Humanes Immunodefizienzvirus Typ 1 (HIV-1) und Humanes Immunodefizienzvirus Typ 2 (HIV-2) seriell verdünnte in dem Substanzträger sind, jeder 'c'eine Verwässerung der vorhergehenden Stufe in dem Substanzträger, wobei das Substanzträgers und die Substanz im Verhältnis 1 zu 99 bis 50 zu 50 sind.

4. Ein Rezeptur zur Verwendung gemäß Anspruch 1, wobei die seriell Verwässerung zwischen 1c und 1millionen c schwankt.

5. Ein Prozess zur Herstellung eines Rezeptur nach eines der Ansprüche 1 bis 4, mit den folgenden Schritten:
(i) Beschaffung einer vorbestimmte Menge der Substanz Immunodefizienzvirus Typ 1 (HIV-1),
(ii) Beschaffung einer vorbestimmte Menge der Substanz Immunodefzienzvirus Typ 2 (HIV-2),
(iii) Mischung einer gleichen Quantität an der der Substanzen Immunodefizienz- virus Typ 1 (HIV-1) und Immunodefizienzvirus Typ 2 (HIV-2),
(iv) Verwässerung der Mischung im Schritt (iii) mit einem geeigneten Betrag des Substanzträgers zur Beschaffung einer Primärverdünnung der Potenz 1c,
(v) Seriell Verdünnung mit der Potenzierung, die Primärverdünnung der Potenz 1c im Schritt (iv) mit dem Substanzträger im Verhältnis der Verdünnung ursprünglichen zum Substanzträger im Verhältnis 1 zu 99 bis 50 zu 50 ist, um eine Kombination der verdünnte und potenzierte HIV von 1c bis 1 Million und mehr zu beschaffen.

6. Ein Prozess zur Herstellung eines Rezeptur nach Anspruch 5, wobei die Potenzierung durch einen Anschlag auf einer Flasche mit einer verdünnten Kultur haltend im geschlossene Hand und jeweils die Hand neu auf einem harten Fläche mit regelmäßiger Häufigkeit oder bei Nutzung der kraftvoll Anschlag mit mechanischen Hilfsmitteln, das eine Flasche auf einem harten Fläche anschlagen kann, durchgef ührt wird.

7. Ein Prozess zur Herstellung eines Rezeptur nach Anspruch 6, wobei die Anschläge 10 mal gegeben sind.

8. Ein Prozess zur Herstellung eines Rezeptur nach Anspruch 6 oder 7, wobei die Fläsche mit einer Zubereitung zum Anschlag für Potenzierung eine verstöpselte gesicherte Gasflasche ist.

9. Ein Prozess zur Herstellung eines Rezeptur nach Anspruch 6 bis 8, wobei einen mechanischen Hilfsmitteln zur Ausübung mindestens einer Kraft von 6 Dynen rythmisch mit einer Frequenz von 10 Anschläge im zwei Minuten.

## Revendications

1. Une Formulation médicinale destinée être utilisée dans le traitement de maladies auto-immunes choiies parmi psoriasis et l'arthrite rhumatismale, ladite formulation comprenant l'immunodéficience humaine de type 1 (VIH-1) et l'immunodéficience humaine de type 2 (VIH-2) dilué et potentialisé en série, ladite formulation étant effectuée dans un véhicule choisi parmi un groupe constitué d'eau distillée ou d'alcool éthylique et un mélange de l'eau distillée et l'alcool éthylique.

2. Une formulation destinée être utilisée selon la revendication 1, dans laquelle les substances sont obtenues à partir de sérums, le sang et les liquides organiques des personnes infectées.

3. Une formulation destinée être utilisée selon la revendication 1, dans laquelle chacune des substances diluées en série sont à dilution 1c à partir des substances originales mêlées de l'immunodéficience humaine de type 1 (VIH-1) et de l'immunodéficience humaine de type 2 (VIH-2), chaque 'c' représentant une dilution de l'étape précédente dans un véhicule, où la proportion de substance au véhicule est entre 1:99 et 50:50..

4. Une formulation destinée être utilisée selon la revendication 1, dans laquelle la dilution en série est entre 1c et 1million c.

5. Un procédé de préparation d'une formulation selon les revendications 1 à 4, comprennant des étapes suivantes:
(i) l'obtention d'une quantité prédéterminée de la substance de l'immunodéficience humaine de type 1 (VIH-1)
(ii) l'obtention d'une quantité prédéterminée de la substance de l'immunodéficience humaine de type 2 (VIH-2)
(iii) le mélange d'une quantité égale de l'immunodéficience humaine de type 1 (VIH-1) et de l'immunodéficience humaine de type 2 (VIH-2);
(iv) la dilution du mélange dans l'étape (iii) avec une quantité approprée du véhicule afin d'obtenir une dilution primaire de 1c.
(v) diluer en série avec potentialisation, la dilution primaire à l'activité 1c dans l'étape (iv) avec le véhicule dans une proportion de la dilution originale au véhicule entre 1:99 et 50:50 afin d'obtenir la combinaison de l'immunodéficience humaine (VIH) potentialisé entre 1c et 1 million et plus.

6. Un procédé de préparation d'une formulation selon la revendication 5, dans laquelle la potentialisation est effectuée par frappant une bouteille avec une culture diluée à poing fermée et en frappant le poing à plusieurs reprises sur une surface dure à une fréquence régulière ou par les coups similaire en utilisant un dispositif mécanique, qui peut frapper une bouteille sur une surface dure.

7. Un procédé de préparation d'une formulation selon la revendication 6, dans laquelle les coups étant donné environ 10 fois.

8. Un procédé de préparation d'une formulation selon la revendication 6 ou 7, dans laquelle la bouteille avec la formulation est une bouteille de verre bien fermé et bouchée pour les coups afin de potentialiser.

9. Un procédé de préparation d'une formulation selon la revendication 6 à 8, dans laquelle un dispositif mécanique est utilisée pour exercer de façon rythmique une force d'au moins 6 dynes à une fréquence de 10 coups en deux minutes.
